# EUROPEAN PATENT APPLICATION

(11) **EP 3 257 427 A1**
(43) Date of publication of application: **20.12.2017**
(21) Application number: 15882134.8
(22) Date of filing: 24.09.2015
(51) Int. Cl.: A61B 1/00

(54) **ENDOSCOPE AND ENDOSCOPE SYSTEM COMPRISING SAME**

(30) Priority: 10.02.2015 KR 20150019865
(71) Applicant: Kang, Yoon Sik, Seoul 06718 (KR); Shin, Chung Yoon, Seoul 06718 (KR); Kang, Hyun Suk, Seoul 06718 (KR); Kang, Hyun Yee, Seoul 06718 (KR)
(72) Inventor: Kang, Yoon Sik, Seoul 06718 (KR); Shin, Chung Yoon, Seoul 06718 (KR); Kang, Hyun Suk, Seoul 06718 (KR); Kang, Hyun Yee, Seoul 06718 (KR)
(74) Representative: Gulde & Partner
(86) International application number: PCT/KR2015/010081
(87) International publication number: WO 2016/129771

(57) **Abstract**

The present invention provides an endoscope capable of more precisely observing curves in the large intestine. The endoscope according to one embodiment of the present invention comprises: a front end part in which a forward filming device is installed; a bending part; and an insertion part, wherein the insertion part has at least one groove formed on the side surface thereof. The groove includes a first inclined surface which becomes more downwardly inclined towards the rear portion of the endoscope. The first inclined surface comprises: a rearward filming device facing the rear side of the endoscope; a light part; and a lens cleaning nozzle.

## Description

### TECHNICAL FIELD

The present invention relates to an endoscope, and more particularly, to an endoscope capable of more precisely observing curves in the large intestine, and an endoscope system including the same.

### BACKGROUND

In general, the primary purpose of an endoscopic examination of the large intestine is to detect and remove a colon polyp (a part of the tissue protruding from an internal wall of the large intestine, which should be removed upon detection of the colon polyp) that is a prodromal change of a colon, thereby fundamentally preventing the colon cancer from occurring. Despite the above purpose, however, it has been known that a failure ratio of detection of the polyp in the endoscopic examination of the large intestine is approximately up to 30%.

Whether residual stool remains in the large intestine as a result of insufficient cleaning of the large intestine, a proficiency or sincerity of an examiner, a short examination time and the like are mentioned as causes of a failure of detection of the colon polyp. However, a more fundamental cause of the failure of detection of the polyp results from an anatomical structure of the large intestine.

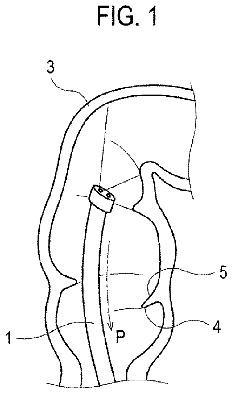

That is, the large intestine 3 is formed with a plurality of curves 4 like the structure of an accordion, as shown in FIG. 1. In the endoscopic examination of the large intestine, after a deep insertion of an endoscope 1 into the large intestine 3, the examiner slowly withdraws the endoscope 1 from the large intestine 3 in a withdrawal direction P while observing the internal wall of the large intestine through a filming device positioned at a front part of the endoscope 1. In such endoscopic examination, however, it is difficult to observe back surfaces 5 of the curves 4 of the internal wall of the large intestine through the filming device provided at the front part of the endoscope 1. Therefore, there is a problem in that it is not easy to detect a polyp formed on the back surfaces 5 of the curves 4.

For this reason, an endoscope of which a bendable angle is increased has been developed to enable the examiner to observe a polyp formed on the back surfaces of the curves by bending the endoscope. However, it is realistically impossible to bend the endoscope at every curve in the large intestine having a small inner diameter for the observation. In addition, there is a high risk that an enterobrosia or the like occurs in the course of bending of the endoscope.

Furthermore, an endoscope in which an additional ring provided with a filming device capable of observing a rear side of the endoscope is installed at a side surface of a front end part of the endoscope, or an endoscope in which a filming device capable of observing a side of the endoscope is installed at a side surface of a front end part of the endoscope has been developed, although these endoscopes are also unsuitable for actual practice.

### SUMMARY

The present invention has been conceived to solve the aforementioned problems of the related art, and an object of the present invention is to provide an endoscope capable of more precisely observing curves in the large intestine, and an endoscope system including the same.

An endoscope according to one embodiment of the present invention has a front end part with a forward filming device installed thereon, a bending part, and an insertion part, wherein at least one groove is formed on a side surface of the front end part. The groove includes a first inclined surface downwardly inclined toward a rear side of the endoscope, and a rearward filming device facing the rear side of the endoscope is installed on the first inclined surface.

An endoscope according to another embodiment of the present invention has a front end part with a forward filming device installed thereon, a bending part, and an insertion part, wherein at least one groove is formed on a side surface of the insertion part. The groove includes a first inclined surface downwardly inclined toward a rear side of the endoscope, and a rearward filming device facing the rear side of the endoscope is installed on the first inclined surface.

An endoscope system according to one embodiment of the present invention includes the endoscope described above.

In a conventional endoscope in which an additional ring provided with a rearward filming device capable of observing a rear side of the endoscope is installed at a side surface of a front end part of the endoscope, the ring protrudes in a lateral direction along the side surface of the front end part of the endoscope so that the diameter of the endoscope in the front end part thereof is increased as much as the thickness of the ring. Accordingly, the likelihood that the rearward filming device may cause pain of a patient upon insertion of the endoscope is increased, and it is very likely that the endoscope may cause an injury to an internal wall of a coelom during the movement of the endoscope in the coelom. Moreover, since the ring as a separate part is installed on the side surface of the front end part of the endoscope, a minute gap is inevitably created between the front end part and the ring, and dregs such as residual stool are trapped in this gap.

Furthermore, in case of a conventional endoscope in which a filming device capable of observing a lateral side of the endoscope is installed on a side surface of a front end part thereof, the filming device is extremely near the internal wall of the large intestine to be observed, whereby a red out phenomenon in which a filmed image is blurred may occur so that it is difficult to perform a precise observation.

On the contrary, the endoscope according to the present invention observes the rear side of the endoscope without employing a separate ring structure. Accordingly, there is an advantageous effect in that it is possible to precisely observe back surfaces of the curves of the large intestine without the problems in the conventional endoscope.

### BRIFE DESCRIPTION OF THE DRAWINGS

FIG. 1 is a view schematically showing a state in which the large intestine is observed by an endoscope.
FIG. 2 shows an endoscope system according to one embodiment of the present invention.
FIG. 3 is a front view of an endoscope according to one embodiment of the present invention.
FIG. 4 is an enlarged perspective view of "A" portion of FIG. 2.
FIG. 5 is a sectional view taken along line V-V of FIG. 4.
FIG. 6 shows another embodiment of a groove formed in a front end part of an endoscope of the present invention.
FIG. 7 is a sectional view taken along line VII-VII of FIG. 6.
FIG. 8 shows an endoscope according to another embodiment of the present invention.
FIG. 9 is an enlarged perspective view of "B" portion of FIG. 8.
FIG. 10 is a sectional view taken along line X-X of FIG. 9.

### DETAILED DESCRIPTION

Hereinafter, embodiments of the present invention will be described in detail with reference to the accompanying drawings. In the following description, however, detailed descriptions of well-known functions and structures will be omitted.

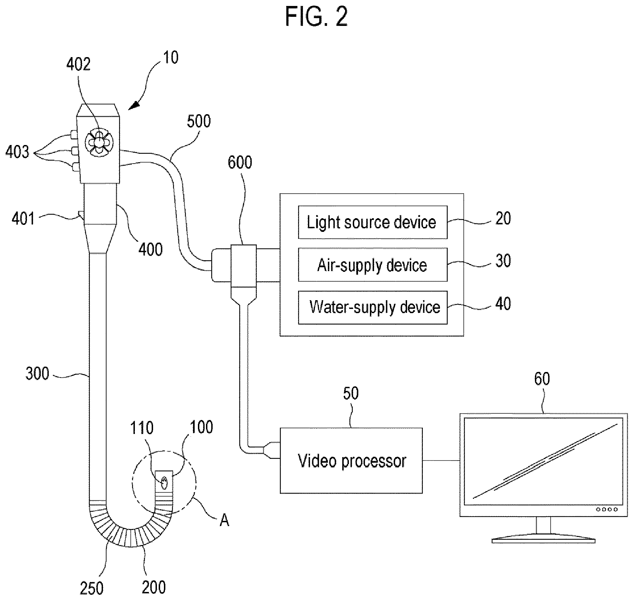

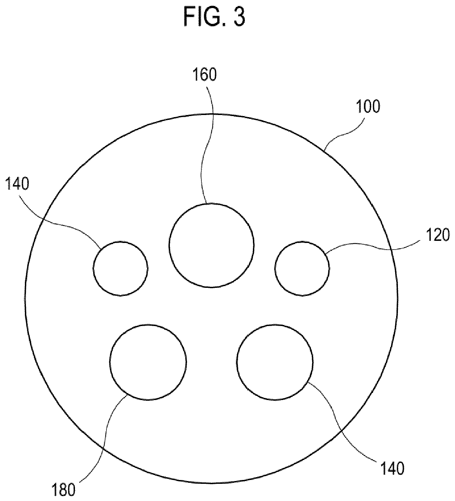

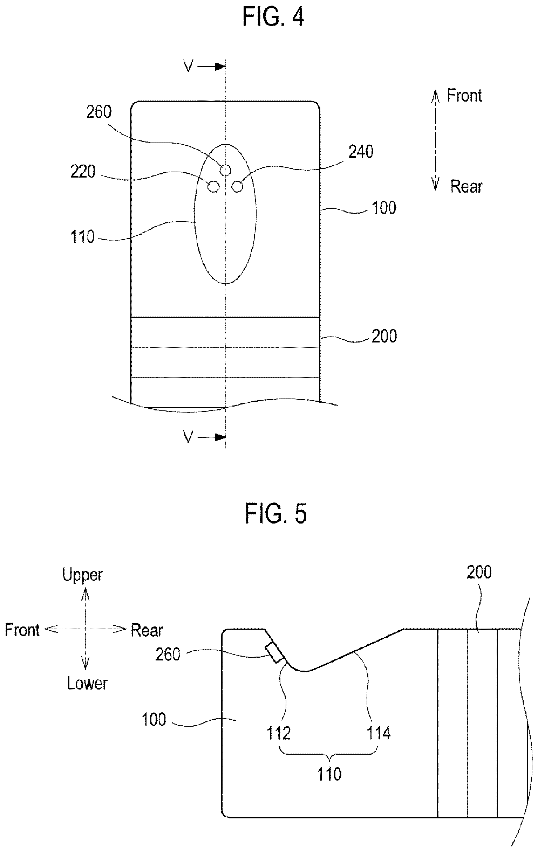

FIG. 2 shows an endoscope system according to one embodiment of the present invention. FIG. 3 is a front view of a front end part 100 of an endoscope 10, and FIG. 4 is an enlarged view of "A" portion of FIG. 2.

For the purpose of explanation, forward and rearward directions are indicated by arrows in FIG. 4, and the directional terms "forward" and "front" used herein mean a direction toward the front end of the endoscope 10 and the directional terms "rearward" and "rear" mean a direction opposite thereto.

Referring to FIGS. 2 and 3, the endoscope system may include the endoscope 10, a light source device 20, an air-supply device 30, a water-supply device 40, a video processor 50 and a monitor 60.

The endoscope 10 is a device inserted into a coelom (for example, large intestine) to observe a biological tissue in the coelom and will be described in detail later. The light source device 20 is a device connected to a connector part 600 of the endoscope 10 and controls a forward light part 140 and a rearward light part 240 installed in the endoscope 100 (see FIG. 4). The video processor 50 is a device connected to the connector part 600 of the endoscope 10, where the video processor receives images are filmed by a forward filming device 160 and a rearward filming device 260 of the endoscope 10, via the connector part 600 and processes the images. The monitor 60 is connected to the video processor 50 and displays the images processed in the video processor 50. The air-supply device 30 and the water-supply device 40 are connected to the connector part 600 of the endoscope 10 to supply air and water, respectively, which are required for washing the endoscope 10.

The endoscope 10 includes the front end part 100, a bending part 200, an insertion part 300, a manipulation part 400, a universal cord 500 and the connector part 600.

The front end part 100 is a part disposed at a foremost end of the endoscope 10. As shown in FIG. 3, a forward lens cleaning nozzle 120 for supplying water and air, the forward light part 140 for radiating light, the forward filming device 160 for filming the interior of the large intestine, and a channel opening 180 through which forceps or the like can be inserted into the large intestine are provided on a front face of the front end part 100.

The forward lens cleaning nozzle 120 is connected to the air-supply device 30 and the water-supply device 40 via the universal cord 500 and the connector part 600 by means of a tube (not shown) within the endoscope 10. Accordingly, water or air is sprayed through the forward lens cleaning nozzle 120 for removing residual stool around the forward filming device 160 or for washing the forward filming device 160.

The forward light part 140 is connected to the light source device 20 via the universal cord 500 and the connector part 600 by means of wiring (not shown) within the endoscope 10. Accordingly, turning-on/off and the like of the forward light part 140 is controlled by the light source device 20 to irradiate a region to be observed.

The forward filming device 160 is connected to the video processor 50 via the universal cord 500 and the connector part 600 by means of wiring (not shown) within the endoscope 10. The actuation of the forward filming device 160 is controlled by the video processor 50 to film the region to be observed, to convert the filmed image into electronic signals and to transmit them to the video processor 50. For example, the forward filming device 160 may include a lens for functioning as an optical system, a solid state imaging device arranged at an image formation location of the lens to film the region to be observed, a correlated double sampling (CDS) circuit for performing a correlated double sampling process for imaging signals created by the solid state imaging device, and an analog/digital conversion circuit for converting analog imaging signals processed in the CDS circuit into digital imaging signals.

The channel opening 180 is connected to an instrument channel 401 extending through the interiors of the front end part 100, the bending part 200, the insertion part 300 and the manipulation part 400, and an instrument such as forceps or the like is inserted through the instrument channel 401 into the large intestine through the channel opening 180.

The bending part 200 is connected to a rear side of the front end part 100 and this bending part 200 serves as a part for causing the front end part 100 to be vertically and horizontally directed depending on the shape and position of the region to be observed. The bending part 200 includes a plurality of bending elements 250 connected to each other, and the bending elements 250 are connected to a bendable wire (not shown) passing through the insertion part 300 and connected to an adjustment knob 402 of the manipulation part 400. As an operator rotates the adjustment knob 402, the bendable wire is pulled or unwound, whereby the bending part 200 can be horizontally and vertically bended.

The insertion part 300 is a part connecting the bending part 200 and the manipulation part 400 and is made of a flexible material. As at least a portion of the insertion part 300 is inserted into or withdrawn from the coelom to move the front end part 100 and the bending part 200 in the coelom.

The manipulation part 400 may include the adjustment knob 402 for adjusting a curvature of the bending part 200, and a control valve 403 provided at tubing through which water and air are supplied into an organ that is being examined, to adjust the supply of air or water. The universal cord 500 is a part for connecting the manipulation part 400 and the connector part 600 and includes a wiring and a tube provided therein. The connector part 600 provides interfaces for connecting the wiring and the tube in the universal cord 500 to the light source device 20, the air-supply device 30, the water-supply device 40 and the video processor 50.

FIG. 4 is an enlarged view of "A" portion of FIG. 2. Referring to FIG. 4, at least one groove 110 is formed on a side surface of the front end part 100 to install the rearward filming device 260 therein. If a plurality of grooves 110 are formed, the grooves 110 of which the number is equal to that of the rearward filming devices 260 to be installed are formed in a circumferential direction of the front end part 100. At this time, it is preferable to form the grooves at a regular angular interval in the circumferential direction. For example, if two grooves 110 are formed, the grooves 110 are formed at an angular interval of 180 degrees in the circumferential direction of the front end part 100. On the other hand, if three grooves 110 are formed, the grooves 110 are formed at an angular interval of 120 degrees in the circumferential direction of the front end part 100. In addition, if four grooves 110 are formed, the grooves 110 are formed at an angular interval of 90 degrees in the circumferential direction of the front end part 100.

FIG. 5 is a sectional view taken along line V-V of FIG. 4. For the purpose of explanation, upward and downward directions are indicated by arrows in FIGS. 5, 7 and 10. As shown in the figures, the directional terms "upward" and "upper" used herein mean a direction in which the groove 110 of the front end part 100 or the insertion part 300 is opened, and the directional terms "downward" and "lower" mean a direction opposite thereto. That is, the directional terms "downward" and "lower" mean a direction from a surface of the front end part 100 or the insertion part 300 toward a center, and the directional terms "upward" and "upper" mean a direction opposite thereto.

As shown in FIG. 5, the groove 110 has a first inclined surface 112 downwardly inclined from a front side to a rear side of the endoscope 10, and a second inclined surface 114 upwardly inclined from the front side to the rear side of the endoscope 10. The rearward filming device 260 is installed on the first inclined surface 112 to be able to observe the rear side of the endoscope 10.

In the present invention, the first inclined surface 112 provides a surface on which the rearward filming device 260 can be installed to face rearward, and the second inclined surface 114 provides a rearward filming range of the rearward filming device 200. The first inclined surface 112 and the second inclined surface 114 of the present invention may be formed as flat or curved surfaces so far as they perform the aforementioned functions.

In a conventional endoscope in which an additional ring provided with a rearward filming device capable of observing a rear side of the endoscope is installed at a side surface of a front end part of the endoscope, the rearward filming device provided on the ring considerably protrudes in a lateral direction along the side surface of the front end part of the endoscope and thus there are the following problems: a diameter of the endoscope at the front end part thereof may be increased as much as a thickness of the ring, the likelihood that the considerably protruding rearward filming device will cause a pain of a patient when the endoscope is being inserted may be increased, and it is very likely that the endoscope may cause an injury to an internal wall of a coelom, such as enterobrosia, during the movement of the endoscope in the coelom. The enterobrosia is an extreme serious complication resulting from the endoscopic examination of the large intestine, and reports show that the enterobrosia occurs at a rate ranging from one case per 6,000 to one case per 500 endoscopic examinations of the large intestine. Although a delicate endoscope-inserting technique is required to prevent the enterobrosia, it is very important to manufacture the endoscope to have a front end structure which does not damage the large intestine. In addition, since the ring as a separate part is installed on the side surface of the front end part of the endoscope, a minute gap is inevitably created between the front end part and the ring, and dregs such as residual stool are trapped in this gap. Therefore, it may be difficult to clean the endoscope after use of the endoscope, and as a result, a risk of cross-infection caused by the endoscope instrument cannot be excluded. Furthermore, since the wiring connected to the rearward filming device is connected to an external power source via the channel opening, a manipulation of forceps through the channel opening for a biopsy may be disturbed upon use of the endoscopic examination, and cleaning of a connecting passage for connecting the channel opening and the instrument channel may be largely disturbed.

On the contrary, in the aforementioned structure according to one embodiment of the present invention, the rearward filming device 260 is installed on the first inclined surface 112 of the groove 110 formed on the side surface of the front end part 110 of the endoscope 10, so that the diameter of the front end part 100 of the endoscope 10 is not increased. In addition, since the structure is not a structure in which an additional part is mounted to the front end part 100 of the endoscope 10, the structure may greatly reduce the likelihood of damaging the wall of the intestine and facilitate cleaning of the endoscope as compared with a conventional structure.

Furthermore, as compared with a conventional endoscope including a lateral filming device capable of observing a lateral side of the endoscope, which is installed on a side surface of a front end part of the endoscope, the aforementioned structure according to one embodiment of the present invention is advantageous in that it is possible to more securely find a polyp. That is, in general, if the filming device is extremely near the internal wall of the large intestine to be observed, a red out phenomenon in which a filmed image is blurred may occur so that it is difficult to perform a precise observation. Therefore, if the lateral filming device provided on the side surface of the front end part of the endoscope observes a region beside the endoscope, the internal wall of the large intestine, which is being observed by the endoscope, becomes extremely near the lateral filming device. On the contrary, in the aforementioned structure according to one embodiment of the present invention, since the rearward filming device 260 is installed on the first inclined surface 112 of the groove 110 formed on the side surface of the front end part 100 of the endoscope 100 so that the rearward filming device 260 faces the rear side of the endoscope 100 (specifically, somewhat slantly faces a rear side of the endoscope), the internal wall of the large intestine observed by the rearward filming device 260 is not a region beside the rearward filming device 260 of the front end part 100 but a region behind the rearward filming device 260. Therefore, since the internal wall of the large intestine which is being observed is far away from the rearward filming device 260, there are advantageous effects in that the likelihood of the red out phenomenon in which the acquired image is blurred is reduced and it is possible to precisely observe back surfaces of the curves.

The rearward lens cleaning nozzle 220 and the rearward light part 240 provided for the rearward filming device 260 are installed in the vicinity of the rearward filming device 260. Similarly to the wiring for the forward filming device 160, the tube for the forward lens cleaning nozzle 120 and the wiring for the forward light part 140 described above, the wiring for the rearward filming device 260, a tube for the rearward lens cleaning nozzle 220 and the wiring for the rearward light part 240 may be connected to the video processor 50, the air and water supply devices 30 and 40 and the light source device 20, respectively, through the universal cord 500 and the connector part 600.

That is, the images filmed by the forward filming device 160 as well as the rearward filming device 260 in the present invention are transmitted to and processed by the video processor 50. Then, information on the processed images is transmitted to and displayed on the monitor 60. It is preferable to simultaneously display the image filmed by the forward filming device 160 and the image filmed by the rearward filming device 260 on an identical screen. In addition, in order to enable the examiner to easily and intuitionally interpret the images, it is preferable to arrange the image filmed by the rearward filming device 260 with respect to the image filmed by the forward filming device 160 in correspondence to the location of the rearward filming device 260. For example, when viewed from the front side of the endoscope, if one of a plurality of rearward filming devices 260 is arranged at a location corresponding to two o'clock, it is preferable to dispose an image filmed by that rearward filming device 260 at a location corresponding to two o'clock with respect to the image filmed by the forward filming device 160.

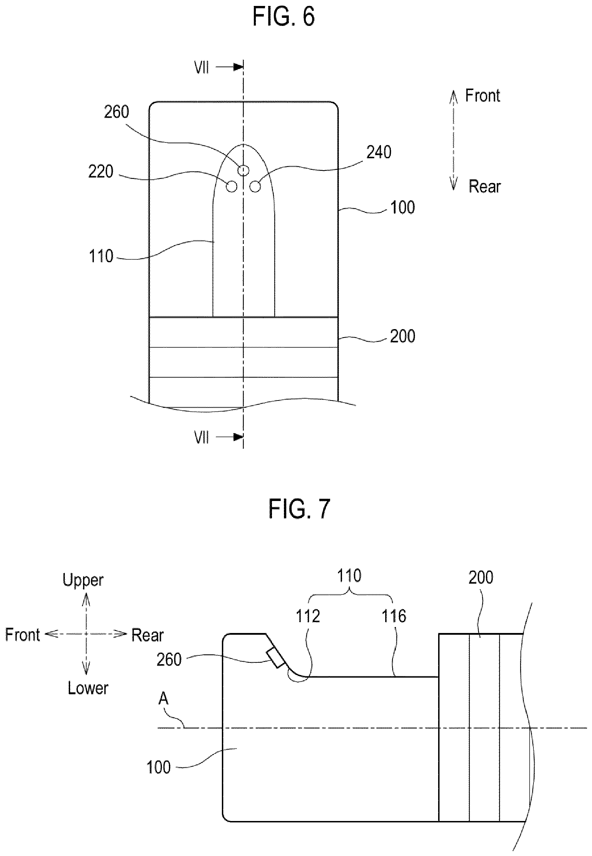

FIG. 6 shows another embodiment of a groove formed on the front end part of the endoscope of the present invention. FIG. 7 is a cross-sectional view taken along line VII-VII of FIG. 6. Referring to FIGS. 6 and 7, the groove 110 according to the other embodiment formed at a front portion of the endoscope of the present invention will be described. The groove 110 is provided with a first inclined surface 112 downwardly inclined from a front side toward a rear side of the endoscope 10 and a bottom surface 116 extending parallel to the axis A of the endoscope 10. The first inclined surface 112 provides a surface on which the rearward filming device 260 facing the rear side can be installed, and the bottom surface 116 extends to the bending part 200 to serve to provide a rear filming range of the rearward filming device 200.

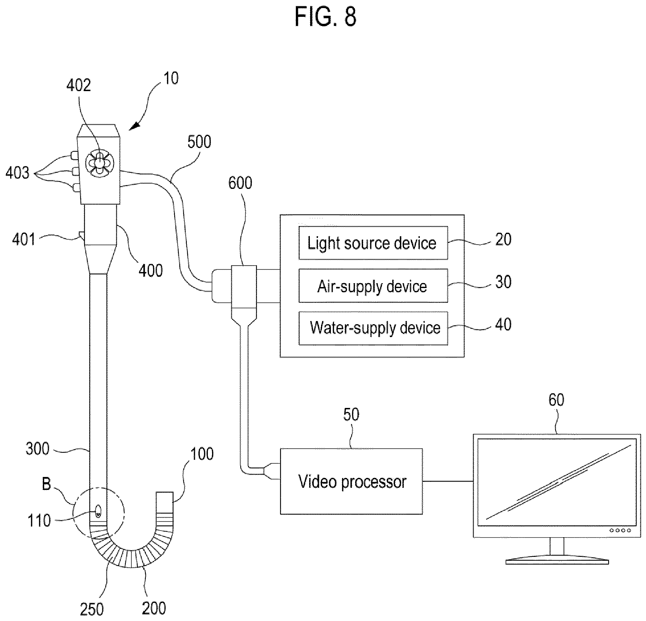

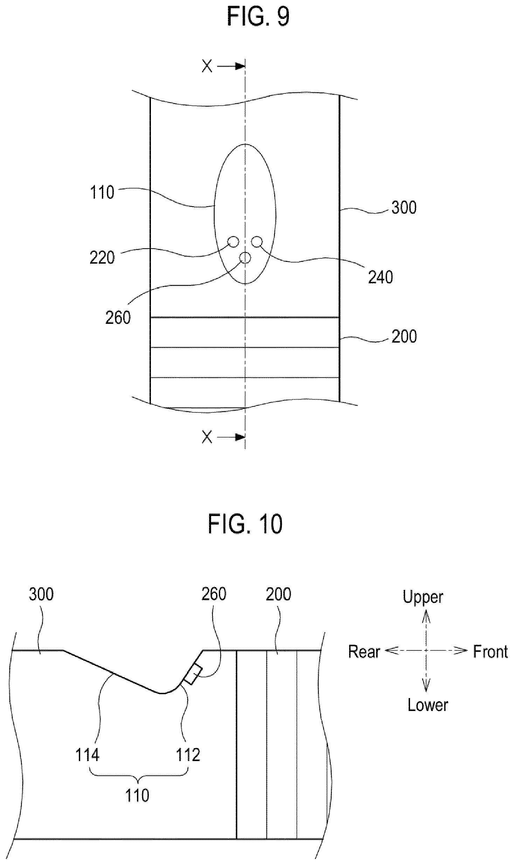

FIG. 8 shows an endoscope according to another embodiment of the present invention. The endoscope shown in FIG. 8 differs from the endoscope shown in FIG. 2 in that the groove 110, the rearward filming device 260, the rearward lens cleaning nozzle 220 and the rearward light part 240 are installed on the insertion part 300 rather than the front end part 100. FIG. 9 is an enlarged view of "B" portion of FIG. 8, and FIG. 10 is a cross-sectional view taken along line X-X of FIG. 9.

As shown in FIGS. 8 to 10, the groove 110 is provided on the insertion part 300. Preferably, the groove is disposed in a portion of the insertion part 300 which is adjacent to the bending part 200. The groove 110 is provided with a first inclined surface 112 downwardly inclined from the front side toward the rear side of the endoscope 10 and a second inclined surface 114 upwardly inclined from the front side toward the rear side of the endoscope 10, and the rearward filming device 260 is provided on the first inclined surface 112 to enable the rearward filming device to observe the rear side of the endoscope 10. Moreover, a rearward lens cleaning nozzle 220 and a rearward light part 240 are installed on the first inclined surface 112 of the groove 110 in the vicinity of the rearward filming device 260.

In this embodiment, there is additional advantage in that the endoscope can consistently observe back surfaces of the curves of the large intestine with a constant sight line irrespective of an operation of the bending part. That is, in a conventional endoscopic examination of the large intestine, after deep insertion of the endoscope into the large intestine, an examiner slowly withdraws the endoscope from the large intestine to observe the internal wall of the large intestine. When a polyp is suspected, the examiner selectively bends the bending part to observe a back surface of a specific curve. According to such an endoscopic examination of the large intestine, if the forward filming device or the rearward filming device is installed at the front end part, the sight line of the forward or rearward filming device is changed by bending the bending part, thereby causing a situation where the examiner cannot be sure whether he/she fully observes the internal wall of the large intestine. In this embodiment, however, regardless of the operation of the bending part, the rearward filming device can observe back surfaces of all curves of the internal wall of the large intestine with the constant sight line as the endoscope is slowly withdrawn from the large intestine.

While the present invention has been described herein in connection with in some embodiments, it should be noted that various changes and modifications may be made without departing from the spirit and scope of the invention which can be understood by those skilled in the art. In addition, it should be considered that such changes and modifications fall within the scope of the claims appended herein.

### DESCRIPTION OF REFERENCE SYMBOLS

100: Front end part, 200: Bending part, 300: Insertion part, 400: Manipulation part, 500: Universal cord, 600: Connector part

## Claims

1. An endoscope, comprising:
a front end part including a forward filming device installed on the front end part; a bending part connected to the front end part and including a plurality of bending elements; an insertion part; and a manipulation part,
wherein the insertion part connects the bending part and the manipulation part,
the front end part and the bending part are moved in a coelom when at least a portion of the insertion part is inserted into or withdrawn from the coelom,
at least one groove is formed on a side surface of the insertion part,
the at least one groove includes a first inclined surface downwardly inclined toward a rear side of the endoscope, and a second inclined surface upwardly inclined toward the rear side of the endoscope, and
a rearward filming device facing the rear side of the endoscope, and a lens cleaning nozzle and a light part for the rearward filming device are installed on the first inclined surface.

2. The endoscope of Claim 1, wherein a plurality of grooves is formed on the side surface of the insertion part, and the plurality of grooves is positioned at a regular angular interval in a circumferential direction of the insertion part.

3. An endoscope system comprising the endoscope of Claim 1.

4. The endoscope system of Claim 3, further comprising a video processor and a monitor, wherein the video processor receives and processes an image filmed by the forward filming device and an image filmed by the rearward filming device, while the monitor simultaneously displays the processed images on an identical screen.

5. The endoscope system of Claim 4, wherein the image filmed by the rearward filming device is arranged on the screen with respect to the image filmed by the forward filming device in correspondence with a location of the rearward filming device.
